# EUROPEAN PATENT SPECIFICATION

(11) **EP 1 689 717 B1**
(45) Date of publication and mention of the grant of the patent: **26.12.2007**
(21) Application number: 04797977.8
(22) Date of filing: 17.11.2004
(51) Int. Cl.: C07D 217/14, C07D 217/22, A61K 31/472, A61P 35/00

(54) **SUBSTITUTED ISOQUINOLINES USEFUL IN THE TREATMENT OF DISEASES SUCH AS CANCER AND ATHEROSCLEROSIS**
FÜR DIE BEHANDLUNG VON KRANKHEITEN WIE KREBS UND ATHEROSCLEROSE GEEIGNETE SUBSTITUIERTE ISOCHINOLINE
ISOQUINOLINES SUBSTITUEES UTILES POUR LE TRAITEMENT DE MALADIES DU TYPE CANCER ET ATHEROSCLEROSE

(30) Priority: 19.11.2003 GB 0326964
(43) Date of publication of application: 16.08.2006
(73) Proprietor: GLAXO GROUP LIMITED, Greenford, Middlesex UB6 ONN (GB)
(72) Inventor: Washio, Yoshiaki, Tsukuba-shi, Ibaraki 300-4247 (JP)
(74) Representative: Giddings, Peter John
(86) International application number: PCT/EP2004/013075
(87) International publication number: WO 2005/049576

(56) References cited:
- WO-A-00/09495
- WO-A1-99/20608
- BARBER, CHRISTOPHER G. ET AL: "Selective urokinase-type plasminogen activator (uPA) inhibitors. Part 3: 1-Isoquinolinylguanidines" BIOORGANIC & MEDICINAL CHEMISTRY LETTERS , 14(12), 3227-3230 CODEN: BMCLE8; ISSN: 0960-894X, 2004, XP002321284

## Description

### BACKGROUND OF THE INVENTION

The present invention relates to isoquinoline derivatives, compositions and medicaments containing the same, as well as processes for the preparation and use of such compounds, compositions and medicaments. Such isoquinoline derivatives are of potential therapeutic benefit in the treatment of diseases associated with inappropriate or pathological angiogenesis.

An important large family of enzymes is the protein kinase enzyme family. Currently, there are about 500 different known protein kinases. Protein kinases serve to catalyze the phosphorylation of an amino acid side chain in various proteins by the transfer of the γ-phosphate of the ATP-Mg²⁺ complex to said amino acid side chain. These enzymes control the majority of the signaling processes inside cells, thereby governing cell function, growth, differentiation and destruction (apoptosis) through reversible phosphorylation of the hydroxyl groups of serine, threonine and tyrosine residues in proteins. Studies have shown that protein kinases are key regulators of many cell functions, including signal transduction, transcriptional regulation, cell motility, and cell division. Several oncogenes have also been shown to encode protein kinases, suggesting that kinases play a role in oncogenesis. These processes are highly regulated, often by complex intermeshed pathways where each kinase will itself be regulated by one or more kinases. Consequently, aberrant or inappropriate protein kinase activity can contribute to the rise of disease states associated with such aberrant kinase activity. Due to their physiological relevance, variety and ubiquitousness, protein kinases have become one of the most important and widely studied family of enzymes in biochemical and medical research.

The protein kinase family of enzymes is typically classified into two main subfamilies: Protein Tyrosine Kinases (PTK) and Protein Serine/Threonine Kinases, based on the amino acid residue they phosphorylate. The serine/threonine kinases (PSTK), includes cyclic AMP- and cyclic GMP-dependent protein kinases, calcium- and phospholipid-dependent protein kinase, calcium- and calmodulin-dependent protein kinases, casein kinases, cell division cycle protein kinases and others. These kinases are usually cytoplasmic or associated with the particulate fractions of cells, possibly by anchoring proteins. Aberrant protein serine/threonine kinase activity has been implicated or is suspected in a number of pathologies such as rheumatoid arthritis, psoriasis, septic shock, bone loss, many cancers and other proliferative diseases. Accordingly, serine/threonine kinases and the signal transduction pathways which they are part of are important targets for drug design. The tyrosine kinases phosphorylate tyrosine residues. Tyrosine kinases play an equally important role in cell regulation. These kinases include several receptors for molecules such as growth factors and hormones, including epidermal growth factor receptor, insulin receptor, platelet derived growth factor receptor and others. Studies have indicated that many tyrosine kinases are transmembrane proteins with their receptor domains located on the outside of the cell and their kinase domains on the inside. Much work is also under progress to identify modulators of tyrosine kinases as well.

The process of angiogenesis is the development of new blood vessels, generally capillaries, from pre-existing vasculature. Angiogenesis is defined as involving (i) activation of endothelial cells; (ii) increased vascular permeability; (iii) subsequent dissolution of the basement membrane and extravisation of plasma components leading to formation of a provisional fibrin gel extracellular matrix; (iv) proliferation and mobilization of endothelial cells; (v) reorganization of mobilized endothelial cells to form functional capillaries; (vi) capillary loop formation; and (vii) deposition of basement membrane and recruitment of perivascular cells to newly formed vessels. Normal angiogenesis is activated during tissue growth, from embryonic development through maturity, and then enters a period of relative quiescence during adulthood. Normal angiogensesis is also activated during wound healing, and at certain stages of the female reproductive cycle. Inappropriate angiogenesis has been associated with several disease states including various retinopathies; ischemic disease; atherosclerosis; chronic inflammatory disorders; and cancer. The role of angiogenesis in disease states is discussed, for instance, in Fan et al, Trends in Pharmacol Sci. 16:54-66; Shawver et al, DDT Vol. 2, No. 2 February 1997; Folkmann, 1995, Nature Medicine 1:27-31.

In cancer the growth of solid tumors has been shown to be angiogenesis dependent. The progression of leukemias as well as the accumulation of fluid associated with malignant ascites and pleural effusions also involve pro-angiogenic factors. (See Folkmann, J., J. Nat'l. Cancer Inst., 1990, 82, 4-6.) Consequently, the targeting of pro-angiogenic pathways is a strategy being widely pursued in order to provide new therapeutics in these areas of great, unmet medical need. The role of tyrosine kinases involved in angiogenesis and in the vascularization of solid tumors has drawn interest.

Until recently most interest in this area has focused on growth factors such as vascular endothelial growth factor (VEGF) and its receptors termed vascular endothelial growth factor receptor(s) (VEGFR). The roles VEGF and VEGFR's play in the vascularisation of solid tumors, progression of hematopoietic cancers and modulation of vascular permeability have drawn great interest in the scientific community. VEGF, a polypeptide, is mitogenic for endothelial cells *in vitro* and stimulates angiogenic responses *in vivo.* VEGF has also been linked to inappropriate angiogenesis (Pinedo, H.M. et al The Oncologist, Vol.5, No. 90001, 1-2, April 2000). VEGFR(s) are protein tyrosine kinases (PTKs). PTKs catalyze the phosphorylation of specific tyrosyl residues in proteins involved in the regulation of cell growth and differentiation. (A.F. Wilks, Progress in Growth Factor Research, 1990, 2, 97-111; S.A. Courtneidge, Dev. Supp.1, 1993, 57-64; J.A. Cooper, Semin. Cell Biol., 1994, 5(6), 377-387; R.F. Paulson, Semin. Immunol., 1995, 7(4), 267-277; A.C. Chan, Curr. Opin. Immunol., 1996, 8(3), 394-401).

Three PTK receptors for VEGF have been identified: VEGFR-1 (Flt-1); VEGFR-2 (Flk-1 or KDR) and VEGFR-3 (Flt-4). These receptors are involved in angiogenesis and participate in signal transduction (Mustonen, T. et al J. Cell Biol. 1995:129:895-898). Of particular interest is VEGFR-2, which is a transmembrane receptor PTK expressed primarily in endothelial cells. Activation of VEGFR-2 by VEGF is a critical step in the signal transduction pathway that initiates tumor angiogenesis. VEGF expression may be constitutive to tumor cells and can also be upregulated in response to certain stimuli. One such stimuli is hypoxia, where VEGF expression is upregulated in both tumor and associated host tissues. The VEGF ligand activates VEGFR-2 by binding with its extracellular VEGF binding site. This leads to receptor dimerization of VEGFRs and autophosphorylation of tyrosine residues at the intracellular kinase domain of VEGFR-2. The kinase domain operates to transfer a phosphate from ATP to the tyrosine residues, thus providing binding sites for signaling proteins downstream of VEGFR-2 leading ultimately to initiation of angiogenesis (McMahon, G., The Oncologist, Vol. 5, No. 90001, 3-10, April 2000).

Angiopoieten 1 (Ang1), a ligand for the endothelium-specific receptor tyrosine kinase TIE-2 is a novel angiogenic factor (Davis et al, Cell, 1996, 87:1161-1169; Partanen et al, Mol. Cell Biol, 12:1698-1707 (1992); U.S. Patent Nos. 5,521,073; 5,879,672; 5,877,020; and 6,030,831). The acronym TIE represents "tyrosine kinase containing lg and EGF homology domains". TIE is used to identify a class of receptor tyrosine kinases, which are exclusively expressed in vascular endothelial cells and early hemopoietic cells. Typically, TIE receptor kinases are characterized by the presence of an EGF-like domain and an immunoglobulin (IG) like domain, which consists of extracellular folding units, stabilized by intra-chain disulfide bonds (Partanen et al Curr. Topics Microbiol. Immunol., 1999, 237:159-172). Unlike VEGF, which functions during the early stages of vascular development, Ang1 and its receptor TIE-2 function in the later stages of vascular development, i.e., during vascular remodeling (remodeling refers to formation of a vascular lumen) and maturation (Yancopoulos et al, Cell, 1998, 93:661-664; Peters, K.G., Circ. Res., 1998, 83(3):342-3; Suri et al, Cell 87, 1171-1180 (1996)).

Consequently, inhibition of TIE-2 would be expected to serve to disrupt remodeling and maturation of new vasculature initiated by angiogenesis thereby disrupting the angiogenic process. Furthermore, inhibition at the kinase domain binding site of VEGFR-2 would block phosphorylation of tyrosine residues and serve to disrupt initiation of angiogenesis. Presumably then, inhibition of TIE-2 and/or VEGFR-2 should prevent tumor angiogenesis and serve to retard or eradicate tumor growth. Accordingly, a treatment for cancer or other disorder associated with inappropriate angiogenesis could be provided.

EphB4 is another receptor tyrosine kinase originally described in J Biol Chem as HTK (1994 May 13;269(19):14211-8) by Bennett BD et al. The recent observation of vascular defects in ephrin-B2 and EphB4 knockout mice strongly suggests that the interaction between the ephrin-B2 ligand and its cognate EphB4 receptor defines the boundaries of arterial-venous domains. Ephrin-B2 ligands are broadly expressed in several other nonvascular tissues such as mesenchymal cells adjacent to vascular endothelial cells, but EphB4 receptors are uniquely localized in vascular endothelial cells. Not only EphB4 receptors are activated by their respective ephrin-B2 ligands, which are also transmembrane proteins, but EphB4 receptors also activate their ephrin-B2 ligands. Embryos heterozygous for EphB4 allele do not show any apparent defects in comparison to wild type. However, homozygous embryos display cardiovascular defects from endothelial cell growth retardation and arrested heart development, and embryonic lethality with high incidence. These results clearly indicate EphB4 signaling pathway plays an essential role in vasculogenesis, angiogenesis and vessel maturation, and these events are also inextricably linked to cancer and atherosclerosis.

The compounds of the present invention possess activities to one or more tyrosine kinases described herein, in particular selected from the group consisting of Tie-2, VEGFR-2, and EphB4 proteins, implicated in cancers or atherosclerosis by inhibiting or preventing inappropriate angiogenesis, vasculogenesis, vessel maturation, or cell motilities. WO 99/20608 and WO00/09495 disclose compounds which inhibit angiogenesis.

### BRIEF SUMMARY OF THE INVENTION

In one aspect of the present invention, there is provided a compound of Formula (1): 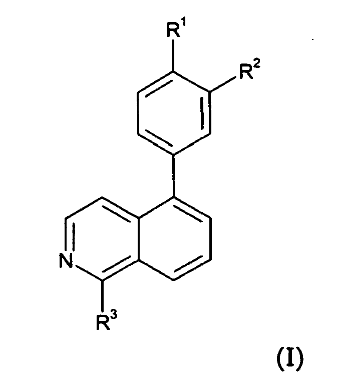 or a salt or a solvate, thereof:
wherein:
One of R¹ and R² is H and the other represents - NHCONHR⁴
wherein R⁴ represents a phenyl or naphthyl group (which may be optionally substituted by one or more substituents independently selected from -C₁₋₆ alkyl, -C₁₋₆ haloalkyl, - CH₂CH₂CH₂-, halogen, C₁₋₆ alkoxy, C₁₋₆ haloalkoxy, OH, NO₂), C₃₋₇ cycloalkyl or R⁴ together with the NH to which it is bonded forms a morpholino group and
R³ is H or NHR⁵ wherein R⁵ is H, -quinolinyl or -isoquinolinyl, -(CONH)p phenyl (wherein p is 0 or 1 and the phenyl is optionally substituted by one or more substituents independently selected from halogen, -C₁₋₆ alkyl, -C₁₋₆ haloalkyl, -morpholino, -SO₂NH₂, benzothiazole (optionally substituted by methyl)).

In an second aspect of the present invention, there is provided a pharmaceutical composition comprising a compound of formula (I), or a salt or a solvate thereof and one or more of pharmaceutically acceptable carriers, diluents and excipients.

In a third aspect of the present invention, there is provided a compound of formula (I), or a salt or a solvate thereof for use in therapy.

There is provided a method of treating a disorder in a mammal, said disorder being mediated by at least one of inappropriate TIE-2, Eph B4 and VEGFR-2 activity, comprising administering to said mammal a compound of formula (I) or a salt, solvate or a physiologically functional derivative thereof (not forming part of the invention).

There is provided the use of a compound of formula (I), or a salt or a solvate, thereof in the manufacture of a medicament for use in the treatment of a disorder mediated by at least one of inappropriate TIE-2, EphB4 and VEGFR-2 activity.

There is provided a method of treating a disorder in a mammal, said disorder being mediated by at least one of inappropriate TIE-2, Eph B4 and VEGFR-2 activity, comprising: administering to said mammal (i) a compound of formula (I), or a salt, solvate or physiologically functional derivative thereof and (ii) an agent to inhibit growth factor receptor function (not forming part of the invention).

There is provided the use of a compound of formula (1) or a salt or a solvate thereof and an agent to inhibit growth factor receptor function in the manufacture of a medicament for the treatment of a disorder mediated by at least one of inappropriate TIE-2, EphB4 and VEGFR2 activity.

There is provided a method of treating a disorder in a mammal, said disorder being characterized by inappropriate angiogenesis, comprising: administering to said mammal a compound of formula (I), or a salt, solvate or physiologically functional derivative thereof (not forming part of the invention).

In a fourth aspect of the invention there is provided the use of a compound of formula (1) or a salt, solvate or physiologically functional derivative thereof in the manufacture of a medicament for the treatment of inappropriate angiogenesis.

### DETAILED DESCRIPTION OF THE INVENTION

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

As used herein, the term "alkyl" refers to a straight or branched chain hydrocarbon radical having the specified number of carbon atoms.. Examples of "alkyl" as used herein include, but are not limited to, methyl, ethyl, n-propyl, isopropyl, n-butyl, isobutyl, t-butyl, n-pentyl, isopentyl, and the like. As used herein, the term "C₁-C₆ alkyl" refers to an alkyl group as defined above containing at least 1, and at most 6, carbon atoms. Examples of branched or straight chained "C₁-C₆ alkyl" groups useful in the present invention include, but are not limited to, methyl, ethyl, n-propyl, isopropyl, isobutyl, n-butyl, t-butyl, n-pentyl, and isopentyl.

As used herein, the term "halogen" refers to fluorine (F), chlorine (Cl), bromine (Br), or iodine (I) and the term "halo" refers to the halogen radicals fluoro (-F), chloro (-CI), bromo (-Br), and iodo (-I).

As used herein, the term "C₁-C₆ haloalkyl" refers to an alkyl group as defined above containing at least 1, and at most 6, carbon atoms substituted with at least one halo group, halo being as defined herein. Examples of branched or straight chained "C₁-C₆ haloalkyl" groups useful in the present invention include, but are not limited to, methyl, ethyl, propyl, isopropyl, isobutyl and n-butyl substituted independently with one or more halos, e.g., fluoro, chloro, bromo and iodo.

As used herein, the term "C₃-C₇ cycloalkyl" refers to a non-aromatic cyclic hydrocarbon ring having from three to seven carbon atoms Exemplary "C₃-C₇ cycloalkyl" groups include, but are not limited to, cyclopropyl, cyclobutyl, cyclopentyl, cyclohexyl and cycloheptyl.

As used herein, the term "alkoxy" refers to the group RₐO-, where Rₐ is alkyl as defined above and the term "C₁-C₆ alkoxy" refers to an alkoxy group as defined herein wherein the alkyl moiety contains at least 1, and at most 6, carbon atoms. Exemplary C₁-C₆ alkoxy groups useful in the present invention include, but are not limited to, methoxy, ethoxy, n-propoxy, isopropoxy, n-butoxy, and t-butoxy.

As used herein, the term "haloalkoxy" refers to the group RₐO-, where Rₐ is haloalkyl as defined above and the term "C₁-C₆ haloalkoxy" refers to a haloalkoxy group as defined herein wherein the haloalkyl moiety contains at least 1, and at most 6, carbon atoms. Exemplary C₁-C₆ haloalkoxy groups useful in the present invention include, but is not limited to, trifluoromethoxy.

As used herein, the term "optionally" means that the subsequently described event(s) may or may not occur, and includes both event(s), which occur, and events that do not occur.

As used herein, the term "physiologically functional derivative" refers to any pharmaceutically acceptable derivative of a compound of the present invention, for example, an ester or an amide, which upon administration to a mammal is capable of providing (directly or indirectly) a compound of the present invention or an active metabolite thereof. Such derivatives are clear to those skilled in the art, without undue experimentation, and with reference to the teaching of Burger's Medicinal Chemistry And Drug Discovery, 5th Edition, Vol 1: Principles and Practice, which is incorporated herein by reference to the extent that it teaches physiologically functional derivatives.

As used herein, the term "solvate" refers to a complex of variable stoichiometry formed by a solute (in this invention, a compound of formula (I) or a salt or physiologically functional derivative thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include, without limitation, water, ethanol and acetic acid. Most preferably the solvent used is water.

As used herein, the term "substituted" refers to substitution with the named substituent or substituents, multiple degrees of substitution being allowed unless otherwise stated.

As used herein, "a compound of the invention" means a compound of formula (I) or a salt or a solvate thereof.

In one embodiment R⁴ represents a phenyl group (which may be optionally substituted by one or more substituents independently selected from -C₁₋₆ haloalkyl, -CH₂CH₂CH₂-, halogen) or C₃₋₇ cycloalkyl.

In one embodiment R³ is H or NHR⁵ wherein R⁵ is H, -quinolinyl, -(CONH)ₚ phenyl (wherein p is 0 or 1 and the phenyl is optionally substituted by one or more substituents independently selected from halogen, -C₁₋₆ haloalkyl, -morpholino, -SO₂NH₂, benzothiazole (subsitituted by methyl)).

Preferably the compound is of formula (1a) : 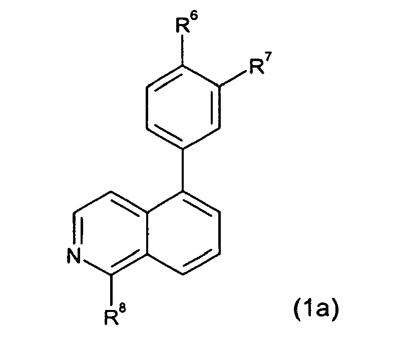 wherein
One of R⁶ and R⁷ is H and the other represents -NHCONHR⁹
wherein R⁹ represents a phenyl group (each of which may be optionally substituted by one or more substituents independently selected from -C₁₋₆ haloalkyl, -CH₂CH₂CH₂-, halogen) or C₃₋₇ cycloalkyl.
R⁸ is H or NHR¹⁰ wherein R¹⁰ is H, -quinolinyl, -(CONH)p phenyl (wherein p is 0 or 1 and the phenyl is optionally substituted by one or more substituents independently selected from halogen, -C₁₋₆ haloalkyl, -morpholino, -SO₂NH₂, benzothiazole substituted by methyl).

Preferably R⁹ represents phenyl, disubstituted with fluoro and trifluoromethyl.

More preferably NHCONHR⁹ is:

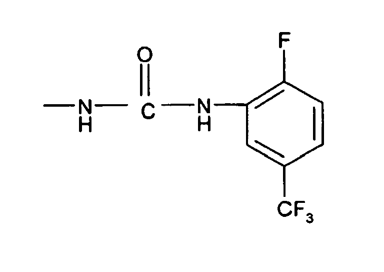

Preferably R¹⁰ is H,

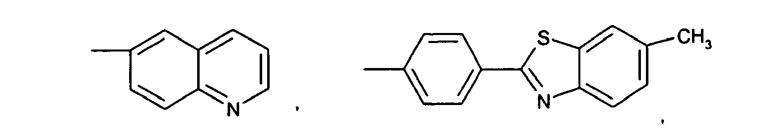

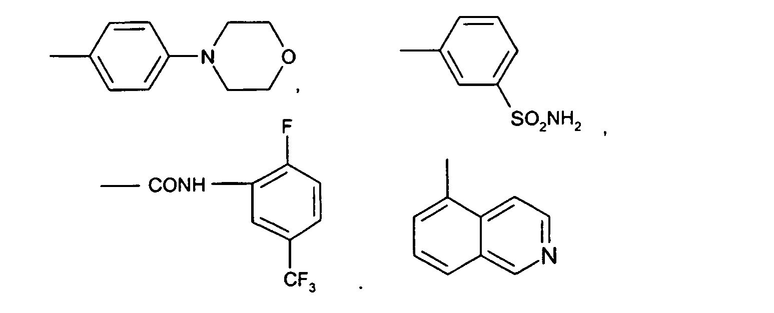

Specific examples of compounds of the present invention include:
1-(2-Fluoro-5-trifluoromethyl-phenyl)-3-(3-isoquinolin-5-ylphenyl)urea;
1-Cyclohexyl-3-(3-isoquinolin-5-ylphenyl)urea;
1-[3-(1-Amino-isoquinolin-5-yl)-phenyl]-3-(2-fluoro-5-trifluoromethyl-phenyl)-urea ;
1-(2-fluoro-5-trifluoromethyl-phenyl)-3-(5-{3-[3-(2-fluoro-5-trifluoromethyl-phenyl)-ureido]-phenyl}-isoquinolin-1-yl)-urea;
1-(2-Fluoro-5-trifluoromethyl-phenyl)-3-{3-[1-(quinolin-6-ylamino)-isoquinolin-5-yl]-phenyl}-urea;
1-(2-Fluoro-5-trifluoromethyl-phenyl)-3-(4-{1-[4-(6-methyl-benzothiazol-2-yl)-phenylamino]-isoquinolin-5-yl}-phenyl)-urea;
1-(2-Fluoro-5-trifluoromethyl-phenyl)-3-(3-{1-[4-(6-methyl-benzothiazol-2-yl)-phenylamino]-isoquinolin-5-yl}-phenyl)-urea;
1-(2-Fluoro-5-trifluoromethyl-phenyl)-3-(4-isoquinolin-5-ylphenyl)urea; 1-Indan-5-yl-3-(3-isoquinolin-5-yl-phenyl)-urea;
1-(2-Fluoro-5-trifluoromethyl-phenyl)-3-{3-[1-(4-morpholin-4-yl-phenylamino)-isoquinolin-5-yl]-phenyl}-urea;
3-{5-[3-(3-Cyclohexyl-ureido)-phenyl]-isoquinolin-1-ylamino}-benzenesulfonamide;

Typically, the salts of the present invention are pharmaceutically acceptable salts. Salts encompassed within the term "pharmaceutically acceptable salts" refer to non-toxic salts of the compounds of this invention. Salts of the compounds of the present invention may comprise acid addition salts derived from a nitrogen on a substituent in the compound of formula (I). Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate. Other salts, which are not pharmaceutically acceptable, may be useful in the preparation of compounds of this invention and these form a further aspect of the invention.

While it is possible that, for use in therapy therapeutically effective amounts of a compound of formula (I), as well as salts and solvates derivatives thereof, may be administered as the raw chemical, it is possible to present the active ingredient as a pharmaceutical composition. Accordingly, the invention further provides pharmaceutical compositions, which include therapeutically effective amounts of compounds of the formula (I) and salt and solvates derivatives thereof, and one or more pharmaceutically acceptable carriers, diluents, or excipients. The compounds of the formula (I) and salts and solvates thereof, are as described above. The carrier(s), diluent(s) or excipient(s) must be acceptable in the sense of being compatible with the other ingredients of the formulation and not deleterious to the recipient thereof. In accordance with another aspect of the invention there is also provided a process for the preparation of a pharmaceutical composition including admixing a compound of the formula (I), or salts and solvates thereof, with one or more pharmaceutically acceptable carriers, diluents or excipients.

Pharmaceutical compositions may be presented in unit dose forms containing a predetermined amount of active ingredient per unit dose. Such a unit may contain, for example, 0.5mg to 1g, preferably 1mg to 700mg, more preferably 5mg to 100mg of a compound of the formula (I), depending on the condition being treated, the route of administration and the age, weight and condition of the patient, or pharmaceutical compositions may be presented in unit dose forms containing a predetermined amount of active ingredient per unit dose. Preferred unit dosage compositions are those containing a daily dose or sub-dose, as herein above recited, or an appropriate fraction thereof, of an active ingredient. Furthermore, such pharmaceutical compositions may be prepared by any of the methods well known in the pharmacy art.

Pharmaceutical compositions may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by bringing into association the active ingredient with the carrier(s) or excipient(s).

Pharmaceutical compositions adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture, as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with a free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit compositions for oral administration can be microencapsulated. The formulation can also be prepared to prolong or sustain the release as for example by coating or embedding particulate material in polymers, wax or the like.

The compounds of formula (I), and salts, solvates and physiological functional derivatives thereof, can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of formula (I) and salts and solvates thereof may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamide -phenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical compositions adapted for topical administration may be formulated as ointments, creams, suspensions, lotions, powders, solutions, pastes, gels, sprays, aerosols or oils.

For treatments of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical compositions adapted for topical administrations to the eye include eye drops wherein the active ingredient is dissolved or suspended in a suitable carrier, especially an aqueous solvent.

Pharmaceutical compositions adapted for topical administration in the mouth include lozenges, pastilles and mouth washes.

Pharmaceutical compositions adapted for rectal administration may be presented as suppositories or as enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical compositions adapted for administration by inhalation include fine particle dusts or mists, which may be generated by means of various types of metered, dose pressurised aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solutions which may contain antioxidants, buffers, bacteriostats and solutes which render the composition isotonic with the blood of the intended recipient; and aqueous and non-aqueous sterile suspensions which may include suspending agents and thickening agents. The compositions may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the compositions may include other agents conventional in the art having regard to the type of formulation in question, for example those suitable for oral administration may include flavouring agents.

A therapeutically effective amount of a compound of the present invention will depend upon a number of factors including, for example, the age and weight of the animal, the precise condition requiring treatment and its severity, the nature of the formulation, and the route of administration, and will ultimately be at the discretion of the attendant physician or veterinarian However, an effective amount of a compound of formula (I) for the treatment of neoplastic growth, for example colon or breast carcinoma, will generally be in the range of 0.1 to 100 mg/kg body weight of recipient (mammal) per day and more usually in the range of 1 to 10 mg/kg body weight per day. Thus, for a 70kg adult mammal, the actual amount per day would usually be from 70 to 700 mg and this amount may be given in a single dose per day or more usually in a number (such as two, three, four, five or six) of sub-doses per day such that the total daily dose is the same. An effective amount of a salt or solvate thereof, may be determined as a proportion of the effective amount of the compound of formula (I) *per se*. It is envisaged that similar dosages would be appropriate for treatment of the other conditions referred to above.

The compounds of the present invention and their salts and solvates, thereof, may be employed alone or in combination with other therapeutic agents for the treatment of the above-mentioned conditions. In particular, in anti-cancer therapy, combination with other chemotherapeutic, hormonal or antibody agents is envisaged as well as combination with surgical therapy and radiotherapy. Combination therapies according to the present invention thus comprise the administration of at least one compound of formula (I) or a pharmaceutically acceptable salt or solvate thereof, and the use of at least one other cancer treatment method. Preferably, combination therapies according to the present invention comprise the administration of at least one compound of formula (I) or a pharmaceutically acceptable salt or solvate thereof, and at least one other pharmaceutically active agent, preferably an anti-neoplastic agent. The compound(s) of formula (I) and the other pharmaceutically active agent(s) may be administered together or separately and, when administered separately this may occur simultaneously or sequentially in any order. The amounts of the compound(s) of formula (I) and the other pharmaceutically active agent(s) and the relative timings of administration will be selected in order to achieve the desired combined therapeutic effect.

The compounds of the Formula (I) or salt or solvates thereof and at least one additional cancer treatment therapy may be employed in combination concomitantly or sequentially in any therapeutically appropriate combination with such other anti-cancer therapies. In one embodiment, the other anti-cancer therapy is at least one additional chemotherapeutic therapy including administration of at least one anti-neoplastic agent. The administration in combination of a compound of formula (I) or salts or solvates thereof with other anti-neoplastic agents may be in combination in accordance with the invention by administration concomitantly in (1) a unitary pharmaceutical composition including both compounds or (2) separate pharmaceutical compositions each including one of the compounds. Alternatively, the combination may be administered separately in a sequential manner wherein one anti-neoplastic agent is administered first and the other second or vice versa. Such sequential administration may be close in time or remote in time.

Anti-neoplastic agents may induce anti-neoplastic effects in a cell-cycle specific manner, i.e., are phase specific and act at a specific phase of the cell cycle, or bind DNA and act in a non cell-cycle specific manner, i.e., are non-cell cycle specific and operate by other mechanisms.

Anti-neoplastic agents useful in combination with the compounds and salts or solvates thereof of formula I include the following:
(1) cell cycle specific anti-neoplastic agents include, but are not limited to, diterpenoids such as paclitaxel and its analog docetaxel; vinca alkaloids such as vinblastine, vincristine, vindesine, and vinorelbine; epipodophyllotoxins such as etoposide and teniposide; fluoropyrimidines such as 5-fluorouracil and fluorodeoxyuridine ; antimetabolites such as allopurinol, fludurabine, methotrexate, cladrabine, cytarabine, mercaptopurine and thioguanine; and camptothecins such as 9-amino camptothecin, irinotecan, topotecan, CPT-11 and the various optical forms of 7-(4-methylpiperazino-methylene)-10,11-ethylenedioxy-20-camptothecin;
(2) cytotoxic chemotherapeutic agents including, but not limited to, alkylating agents such as melphalan, chlorambucil, cyclophosphamide, mechlorethamine, hexamethylmelamine, busulfan, carmustine, lomustine, and dacarbazine; anti-tumour antibiotics such as doxorubicin, daunomycin, epirubicin, idarubicin, mitomycin-C, dacttinomycin and mithramycin; and platinum coordination complexes such as cisplatin, carboplatin, and oxaliplatin; and
(3) other chemotherapeutic agents including, but not limited to, anti-estrogens such as tamoxifen, toremifene, raloxifene, droloxifene and iodoxyfene; progestrogens such as megestrol acetate; aromatase inhibitors such as anastrozole, letrazole, vorazole, and exemestane; antiandrogens such as flutamide, nilutamide, bicalutamide, and cyproterone acetate; LHRH agonists and antagagonists such as goserelin acetate and luprolide, testosterone 5α-dihydroreductase inhibitors such as finasteride; metalloproteinase inhibitors such as marimastat; antiprogestogens; urokinase plasminogen activator receptor function inhibitors; growth factor function inhibitors such as inhibitors of the functions of hepatocyte growth factor; erb-B2, erb-B4, epidermal growth factor receptor (EGFR), platelet derived growth factor receptor (PDGFR), vascular endothelial growth factor receptor (VEGFR, and EpHB4, TIE-2 (other than those VEGFR, EpHB4 and TIE-2 inhibitors described in the present invention); and other tyrosine kinase inhibitors such as inhibitors of CDK2 and CDK4 inhibitors.

The compounds of formula (I) and salt or solvates thereof, are believed to have anticancer activity as a result of inhibition of the protein kinase TIE-2, EpHB4 and/or VEGFR-2 and its effect on selected cell lines whose growth is dependent on TIE-2, EpHb B4 and/or VEGFR-2 protein kinase activity.

The present invention thus also provides compounds of formula (I) and pharmaceutically acceptable salts or solvates thereof, for use in medical therapy, and particularly in the treatment of disorders mediated by at least one of inappropriate TIE-2, EpHB4 and VEGFR-2 activity.

The inappropriate TIE-2, EpH B4 and/or VEGFR-2 activity referred to herein is any TIE-2 , EpH B4 and/or VEGFR-2 activity that deviates from the normal TIE-2, EpH B4 and/or VEGFR-2 activity expected in a particular mammalian subject. Inappropriate TIE-2, EpH B4 and/or VEGFR-2 activity may take the form of, for instance, an abnormal increase in activity, or an aberration in the timing and or control of TIE-2, EpH B4 and/or VEGFR-2 activity. Such inappropriate activity may result then, for example, from overexpression or mutation of the protein kinase leading to inappropriate or uncontrolled activation. Furthermore, it is also understood that unwanted TIE-2, EpH B4 and/or VEGFR-2 activity may reside in an abnormal source, such as a malignancy. That is, the level of TIE-2, EpH B4 and/or VEGFR-2 activity does not have to be abnormal to be considered inappropriate, rather the activity derives from an abnormal source. In a like manner, the inappropriate angiogenesis referred to herein is any angiogenic activity that deviates from the normal angiogenic activity expected in a particular mammalian subject. Inappropriate angiogenesis may take the form of, for instance, an abnormal increase in activity, or an aberration in the timing and or control of angiogenic activity. Such inappropriate activity may result then, for example, from overexpression or mutation of a protein kinase leading to inappropriate or uncontrolled activation. Furthermore, it is also understood that unwanted angiogenic activity may reside in an abnormal source, such as a malignancy. That is, the level of angiogenic activity does not have to be abnormal to be considered inappropriate, rather the activity derives from an abnormal source.

The present invention is directed to methods of regulating, modulating, or inhibiting TIE-2, EpH B4 and/or VEGFR-2 for the prevention and/or treatment of disorders related to unregulated TIE-2, EpH B4 and/or VEGFR-2 activity. In particular, the compounds of the present invention can also be used in the treatment of certain forms of cancer. Furthermore, the compounds of the present invention can be used to provide additive or synergistic effects with certain existing cancer chemotherapies, and/or be used to restore effectiveness of certain existing cancer chemotherapies and radiation.

The compounds of the present invention are also useful in the treatment of one or more diseases afflicting mammals which are characterized by cellular proliferation in the area of disorders associated with neo-vascularization and/or vascular permeability including blood vessel proliferative disorders including arthritis and restenosis; fibrotic disorders including hepatic cirrhosis and atherosclerosis; mesangial cell proliferative disorders include glomerulonephritis, diabetic nephropathy, malignant nephrosclerosis, thrombotic microangiopathy syndromes, organ transplant rejection and glomerulopathies; and metabolic disorders include psoriasis, diabetes mellitus, chronic wound healing, inflammation and neurodegenerative diseases.

A further aspect is a method of treatment of a mammal suffering from a disorder mediated by at least one of inappropriate TIE-2, EpH B4 and VEGFR-2 activity, including susceptible malignancies, which includes administering to said subject a compound of formula (I) or a pharmaceutically acceptable salt, solvate, or a physiologically functional derivative thereof. In a preferred embodiment, the disorder is cancer (not part of the invention).

A further aspect is a method of treatment of a mammal suffering from cancer which includes administering to said subject a compound of formula (I) or a pharmaceutically acceptable salt or solvate thereof, or a physiologically functional derivative thereof (not part of the invention).

A further aspect is the use of a compound of formula (I), or a pharmaceutically acceptable salt or solvate thereof in the preparation of a medicament for the treatment of a disorder characterized by at least one of inappropriate TIE-2, EpH B4 and VEGFR-2 activity. In a preferred embodiment, the disorder is cancer.

A further aspect of the present invention provides the use of a compound of formula (I), or a pharmaceutically acceptable salt or solvate thereof in the preparation of a medicament for the treatment of cancer and malignant tumors.

The mammal requiring treatment with a compound of the present invention is typically a human being.

In another embodiment, therapeutically effective amounts of the compounds of formula (I) or salts or solvates thereof and agents which inhibit growth factor receptor function may be administered in combination to a mammal for treatment of a disorder mediated by at least one of inappropriate TIE-2 EpHB4 and VEGFR-2 activity, for instance in the treatment of cancer. Such growth factor receptors include, for example, EGFR, PDGFR, erbB2 and erbB4. Growth factor receptors and agents that inhibit growth factor receptor function are described, for instance, in Kath, John C., Exp. Opin. Ther. Patents (2000) 10(6):803-818 and in Shawver et al DDT Vol 2, No. 2 February 1997.

The compounds of the formula (I) or salts or solvates thereof and the agent for inhibiting growth factor receptor function may be employed in combination concomitantly or sequentially in any therapeutically appropriate combination. The combination may be employed in combination in accordance with the invention by administration concomitantly in (1) a unitary pharmaceutical composition including both compounds or (2) separate pharmaceutical compositions each including one of the compounds. Alternatively, the combination may be administered separately in a sequential manner wherein one is administered first and the other second or vice versa. Such sequential administration may be close in time or remote in time.

In another aspect, there is provided a method of treating a disorder in a mammal, said disorder being mediated by inappropriate angiogenesis, including: administering to said mammal a compound of formula (I), or a salt, solvate or physiologically functional derivative thereof. In one embodiment, the inappropriate angiogenic activity is due to at least one of inappropriate VEGFR2, EpHB4, or TIE-2 activity. In another embodiment, the inappropriate angiogenesis is due to inappropriate VEGFR2 and TIE-2 activity. In a further embodiment, the method further includes administering a VEGFR2 inhibitor along with the compounds of formula (I) or salts, solvates or physiologically functional derivatives thereof. Preferably the disorder is cancer (not forming part of the invention).

In another aspect there is provided the use of a compound of formula (I), or a salt or solvate thereof in the preparation of a medicament for use in treating a disorder in a mammal, said disorder being characterized by inappropriate angiogenesis. In one embodiment, the inappropriate angiogenic activity is due to at least one of inappropriate VEGFR2, EpHB4, VEGFR3 or TIE-2 activity. In another embodiment, the inappropriate angiogenesis is due to inappropriate VEGFR2, EpH B4 and TIE-2 activity. In a further embodiment, the use further includes use of a VEGFR2 inhibitor to prepare said medicament.

The combination of a compound of formula (I) or salts or solvates thereof with a VEGFR2 inhibitor may be employed in combination in accordance with the invention by administration concomitantly in (1) a unitary pharmaceutical composition including both compounds or (2) separate pharmaceutical compositions each including one of the compounds. Alternatively, the combination may be administered separately in a sequential manner wherein one is administered first and the other second or vice versa. Such sequential administration may be close in time or remote in time.

The compounds of this invention may be made by a variety of methods, including standard chemistry. Any previously defined variable will continue to have the previously defined meaning unless otherwise indicated. Illustrative general synthetic methods are set out below and then specific compounds of the invention are prepared in the Working Examples.

Compounds of general formula (I) may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (T. W. Green and P. G. M. Wuts (1991) Protecting Groups in Organic Synthesis, John Wiley & Sons). These groups are removed at a convenient stage of the compound synthesis using methods that are readily apparent to those skilled in the art. The selection of processes as well as the reaction conditions and order of their execution shall be consistent with the preparation of compounds of Formula (I). Those skilled in the art will recognize if a stereocenter exists in compounds of Formula (I). Accordingly, the present invention includes both possible stereoisomers and includes not only racemic compounds but the individual enantiomers as well. When a compound is desired as a single enantiomer, it may be obtained by stereospecific synthesis or by resolution of the final product or any convenient intermediate. Resolution of the final product, an intermediate, or a starting material may be effected by any suitable method known in the art. See, for example, Stereochemistry of Organic Compounds by E. L. Eliel, S. H. Wilen, and L. N. Mander (Wiley-Interscience, 1994).

Compounds of Formula (I) can be prepared according to the synthetic sequences depicted generally and by illustrative example below and further illustrated by specific Examples.

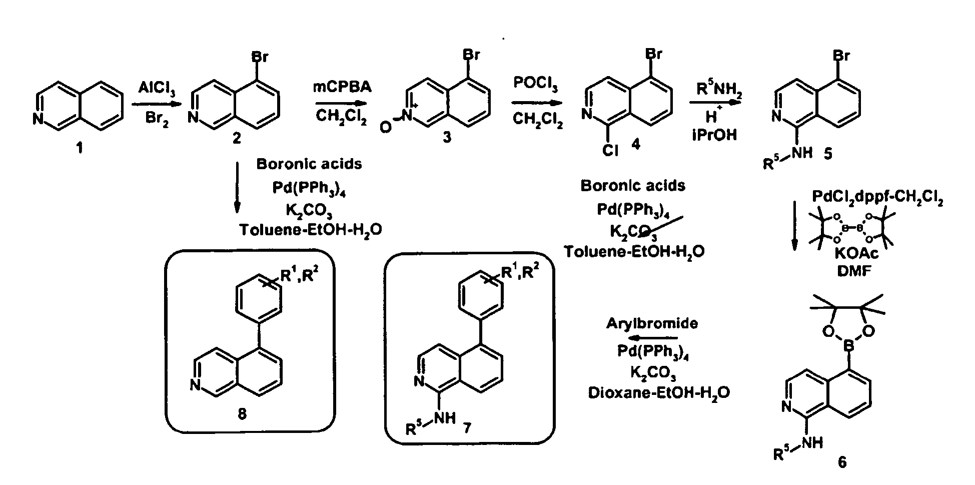

Certain embodiments of the present invention will now be illustrated by way of example only. The physical data given for the compounds exemplified is consistent with the assigned structure of those compounds.

### EXAMPLES

As used herein the symbols and conventions used in these processes, schemes and examples are consistent with those used in the contemporary scientific literature, for example, the *Journal of the American Chemical Society* or the *Journal of Biological Chemistry.* Standard single-letter or three-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:

| | |
|---|---|
| g (grams); | mg (milligrams); |
| L (liters); | mL (milliliters); |
| µL (microliters); | psi (pounds per square inch); |
| M (molar); | mM (millimolar); |
| i. v. (intravenous); | Hz (Hertz); |
| MHz (megahertz); | mol (moles); |
| mmol (millimoles); | rt (room temperature); |
| min (minutes); | h (hours); |
| mp (melting point); | TLC (thin layer chromatography); |
| Tᵣ (retention time); | RP (reverse phase); |
| MeOH (methanol); | i-PrOH (isopropanol); |
| TEA (triethylamine); | TFA (trifluoroacetic acid); |
| TFAA (trifluoroacetic anhydride); | THF (tetrahydrofuran); |
| DMSO (dimethylsulfoxide); | AcOEt (ethyl acetate); |
| DME (1,2-dimethoxyethane); | DCM (dichloromethane); |
| DCE (dichloroethane); | DMF (N,N-dimethylformamide); |
| DMPU (N,N'-dimethylpropyleneurea); | (CDI (1,1-carbonyldiimidazole); |
| IBCF (isobutyl chloroformate); | HOAc (acetic acid); |
| HOSu (N-hydroxysuccinimide); | HOBT (1-hydroxybenzotriazole); |
| mCPBA (meta-chloroperbenzoic acid; | EDC (ethylcarbodiimide hydrochloride); |
| BOC (tert-butyloxycarbonyl); | FMOC (9-fluorenylmethoxycarbonyl); |
| DCC (dicyclohexylcarbodiimide); | CBZ (benzyloxycarbonyl); |
| Ac (acetyl); | atm (atmosphere); |
| TMSE (2-(trimethylsilyl)ethyl); | TMS (trimethylsilyl); |
| TIPS (triisopropylsilyl); | TBS (t-butyldimethylsilyl); |
| DMAP (4-dimethylaminopyridine); | BSA (bovine serum albumin) |
| ATP (adenosine triphosphate); | HRP (horseradish peroxidase); |
| DMEM (Dulbecco's modified Eagle medium); | |
| HPLC (high pressure liquid chromatography); | |
| BOP (bis(2-oxo-3-oxazolidinyl)phosphinic chloride); | |
| TBAF (tetra-n-butylammonium fluoride); | |
| HBTU (O-Benzotriazole-1-yl-N,N,N',N'- tetramethyluronium hexafluorophosphate). | |
| HEPES (4-(2-hydroxyethyl)-1-piperazine ethane sulfonic acid); | |
| DPPA (diphenylphosphoryl azide); | |
| fHNO₃ (fumed HNO₃); and | |
| EDTA (ethylenediaminetetraacetic acid). | |

All references to ether are to diethyl ether; brine refers to a saturated aqueous solution of NaCl. Unless otherwise indicated, all temperatures are expressed in °C (degrees Centigrade). All reactions are conducted under an inert atmosphere at room temperature unless otherwise noted.

¹H NMR spectra were recorded on a Varian VXR-300, a Varian Unity-300, a Varian Unity-400 instrument, a Brucker AVANCE-400, or a General Electric QE-300. Chemical shifts are expressed in parts per million (ppm, δ units). Coupling constants are in units of hertz (Hz). Splitting patterns describe apparent multiplicities and are designated as s (singlet), d (doublet), t (triplet), q (quartet), quint (quintet), m (multiplet), br (broad).

HPLC were recorded on a Gilson HPLC or Shimazu HPLC system by the following conditions. Column: 50 X 4.6mm (id) stainless steel packed with 5µm Phenomenex Luna C-18 ; Flow rate: 2.0 mL/min; Mobile phase: A phase = 50mM ammonium acetate (pH 7.4), B phase = acetonitrile, 0-0.5min (A: 100%, B: 0%), 0.5-3.0 min (A:100-0%, B:0-100%), 3.0-3.5min (A: 0%, B: 100%), 3.5-3.7 min (A: 0-100%, B: 100-0%), 3.7-4.5 min (A: 100%, B: 0%); Detection : UV 254nm; Injection volume: 3µL.

Low-resolution mass spectra (MS) were recorded on a JOEL JMS-AX505HA, JOEL SX-102, or a SCIEX-APliii spectrometer; LC-MS were recorded on a micromass 2MD and Waters 2690; high resolution MS were obtained using a JOEL SX-102A spectrometer. All mass spectra were taken under electrospray ionization (ESI), chemical ionization (Cl), electron impact (EI) or by fast atom bombardment (FAB) methods. Infrared (IR) spectra were obtained on a Nicolet 510 FT-IR spectrometer using a 1-mm NaCl cell. Most of the reactions were monitored by thin-layer chromatography on 0.25 mm E. Merck silica gel plates (60F-254), visualized with UV light, 5% ethanolic phosphomolybdic acid or p-anisaldehyde solution. Flash column chromatography was performed on silica gel (230-400 mesh, Merck).

### Example 1.

### 1-(2-Fluoro-5-trifluoromethyl-phenyl)-3-(3-isoauinolin-5-ylphenyl)urea

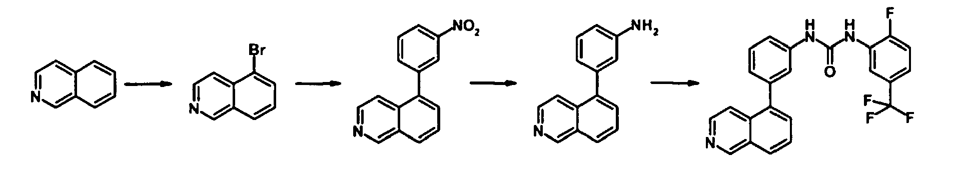

### a. 5-Bromoisoquinoline

To a suspension of AlCl₃ (156.7 g, 1.18 mol) in CH₂Cl₂ (500 mL), a solution of isoquinoline (605 mmol, 71 mL) in CH₂Cl₂ (100 mL) was dropwise added at such rate that the reaction mixture was refluxed gently. After addition, CH₂Cl₂ was removed by distillation. The blackish residue was melted at 120°C then the temperature was adjusted to 100°C. To the mixture, Br2 (31 mL, 605 mmol) was dropwise added over 2hrs at 100°C and stirred for 30min at same temperature, then was stirred at 75°C overnight. The mixture was cooled to RT then carefully poured into ice-water. The aqueous mixture was basified with NaOHaq. and extracted with ether. The organics was dried over Na₂SO₄ then evaporated. Sequence purification on SiO₂ column chromatography twice and recrystalisation (from hexane) gave the title compound (34.5 g, 28%).

### b. 5-(3-Nitrophenyl)isoquinoline

A mixture of 5-bromoisoquinoline (4.16 g, 20 mmol), Pd(PPh₃)4 (290 mg, 0.25 mmol) and 3-nitrophenylboronic acid (3.67 g, 22 mmol) was flushed N₂ gas then added dioxane (200 mL), ethanol (40 mL) and 2M K2C03aq (200 mL). The mixture was stirred at 100°C overnight. After cooling, the mixture was evaporated to remove dioxane then extracted with AcOEt. The organic layer was washed with brine then dried over Na₂SO₄ After evaporation, the residue was purified on SiO₂ column chromatography to give the title compound (4.65 g, 93%).

### c. 5-(3-Aminophenyl)isoguinoline

To a solution of 5-(3-nitrophenyl)isoquinoline (751 mg, 3 mmol), Zn powder (ca 1 g) was added and stirred at room temperature overnight. Insoluble materials were removed by filtration then evaporated to remove solvent. The residue was extracted with AcOEt. The organic layer was washed with NaHCO₃aq. and brine then dried over Na₂SO₄. After evaporation, the residue was purified on SiO₂ column chromatography and on SCX-SPE to give the title compound (521 mg, 79%).

### d. 1-(2-Fluoro-5-trifluoromethyl-phenyl)-3-(3-isoquinolin-5-ylphenyl)urea

To a solution of 5-(3-aminophenyl)isoquinoline (30 mg, 0.136 mmol) in THF (1.5mL), 2-fluoro-5-(trifluoromethyl)phenyl isocyanate (24µL, 0.16 mmol) was added then stirred at room temperature for 3 days. The mixture was purified on SCX-SPE then formed solid was washed with MeOH to give the title compound (61.2 mg, 98%).

### Example 2.

### -Cyclohexyl-3-(3-isoguinolin-5-ylphenyl)urea

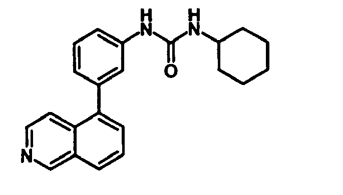

The title compound was prepared from 5-(3-aminophenyl)isoquinoline and cyclohexyl isocyanate as described in example 1d.

### Example 3 and 4.

### 1-[3-(1-Amino-isoquinolin-5-yl)-phenyl]-3-(2-fluoro-5-trifluoromethyl-phenyl)-urea (Example 3) and 1-(2-fluoro-5-trifluoromethyl-phenyl)-3-(5-{3-[3-(2-fluoro-5-trifluoromethyl-phenyl)-ureido]-phenyl}-isoquinolin-1-yl)-urea (Example 4)

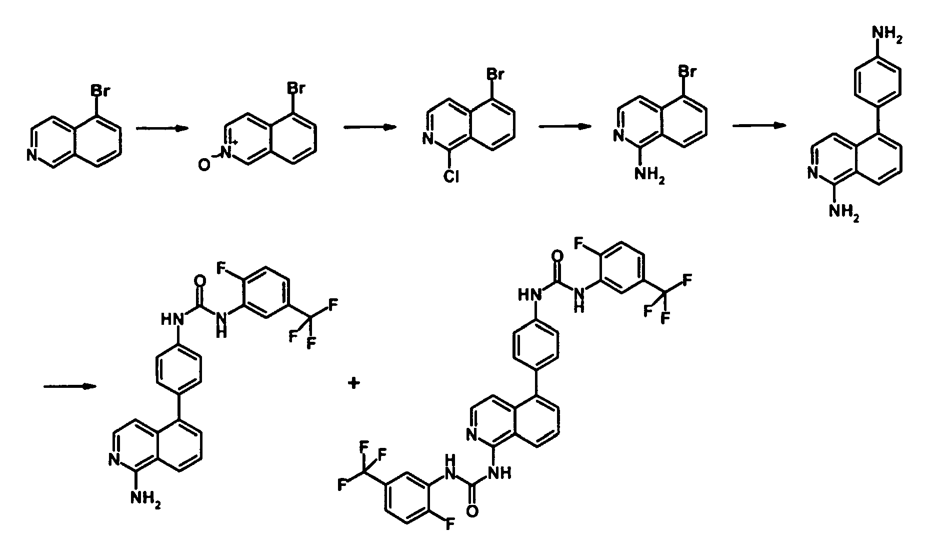

### a. 5-Bromoisoquinoline N-oxide

To a solution of 5-bromoisoquinoline (20.8 g, 100 mmol) in CH₂Cl₂ (500 mL), mCPBA (80 % assay, 23.7 g, 110 mmol) was added and stirred at 45°C overnight. After cooling, the mixture was quenched with Na₂S₂O₃ then extracted with CH₂Cl₂-The organic layer was washed with NaOHaq., dried over Na₂SO₄ then evaporated. Sequence recrystalisation (from CH₂Cl₂-ether) gave the title compound (20.1 g, 90 %).

### b. 5-Bromo-l-chloroisoquinoline

To a solution of 5-bromoisoquinoline N-oxide (20.1 g, 89.5 mmol) in CH₂Cl₂ (500 mL), POCl₃ (20 mL, 215 mmol) was added and stirred at 45°C overnight. After cooling, the mixture was evaporated to remove POCl3 then added water. The mixture was extracted with CH₂Cl₂. The organic layer was washed with NaHCO₃aq., dried over Na₂SO₄ then evaporated. Formed solid was washed with MeOH to give the title compound (16.1 g, 74%).

### c. 1-Amino-5-bromoisoquinoline

A suspension of 5-bromo-1-chloroisoquinoline (2.0 g, 8.25 mmol) in sat. NH₃-MeOH (100 mL) was heated to 180°C for 15days in autoclave. After cooling, the solvent was removed by evapolation. The residue was washed with CH₂Cl₂ then purified on SCX SPE. Formed solid was washed with hexane to give the title compound (1.57 g, 85%).

### d. 1-Amino-5-(4-aminophenyl)isoquinoline

The title compound was prepared from 1-amino-5-bromoisoquinoline and 4-(4,4,5,5-Tetramethyl-[1,3,2]dioxaborolan-2-yl)aniline as described in example 1b.

### e. 1-[3-(1-Amino-isoguinolin-5-yl)-phenyl]-3-(2-fluoro-5-trifluoromethyl-phenyl)-urea and 1-(2-fluoro-5-trifluoromethyl-phenyl)-3-(5-{3-[3-(2-fluoro-5-trifluoromethylPhenyl)-ureido]-phenyl}-isoguinolin-1-yl)-urea

The title compounds were prepared from 1-amino-5-(4-aminophenyl)isoquinoline as described in example 1d as mixture. These compounds were separated by column chromatography.

### Example 5.

### 1-(2-Fluoro-5-trifluoromethyl-phenyl)-3-{3-[1-(quinolin-6-ylamino)-isoquinolin-5-yl]-phenyl}-urea

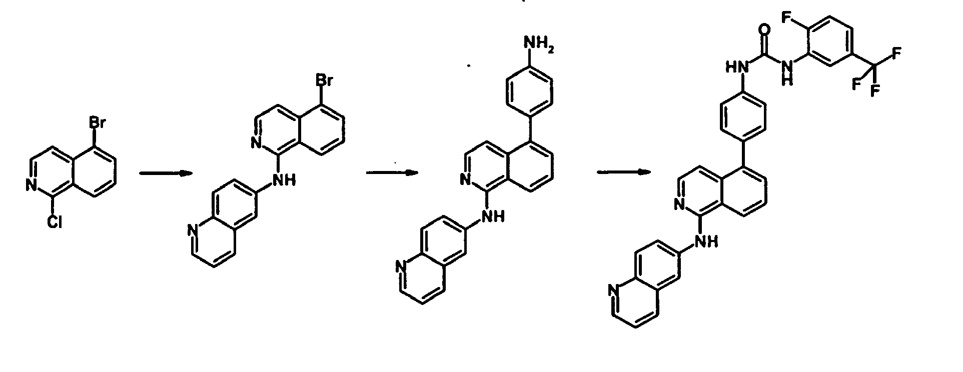

### a. 5-Bromo-1-(quinolin-6-yl)aminoisoquinoline

To a suspension of 5-bromo-1-chloroisoquinoline (3.0 g, 12.4 mmol) in iPrOH (100 mL), 6-aminoquinoline (4.5 g, 31.2 mmol), 4M HCl-dioxane (5 mL) and MeOH (15 mL) were added and stirred at 80°C 3dys. After cooling, the mixture was evaporated to remove solvent then suspended into AcOEt. The mixture was basified with NaHCO₃aq. then formed precipitate was collected by filtration and washed with AcOEt to give the title compound (3.4 g, 78%).

### b. 5-(4-Aminophenyl)-1-(quinolin-6-yl)aminoisoquinoline

The title compound was prepared from 5-bromo-1-(quinolin-6-yl)aminoisoquinoline and 4-(4,4,5,5-Tetramethyl-[1,3,2]dioxaborolan-2-yl)aniline as described in example 1b.

### c. 1-(2-Fluoro-5-trifluoromethyl-phenyl)-3-(3-[1-(quinolin-6-ylamino)-isoquinolin-5-yl]-phenyl}-urea

The title compound was prepared from 5-(4-aminophenyl)-1-(quinolin-6-yl)aminoisoquinoline as described in example 1d.

### Example 6.

### 1-(2-Fluoro-5-trifluoromethyl-phenyl)-3-(4-{1-[4-(6-methyl-benzothiazol-2-yl)-phenylamino]-isoquinolin-5-yl}-phenyl)-urea

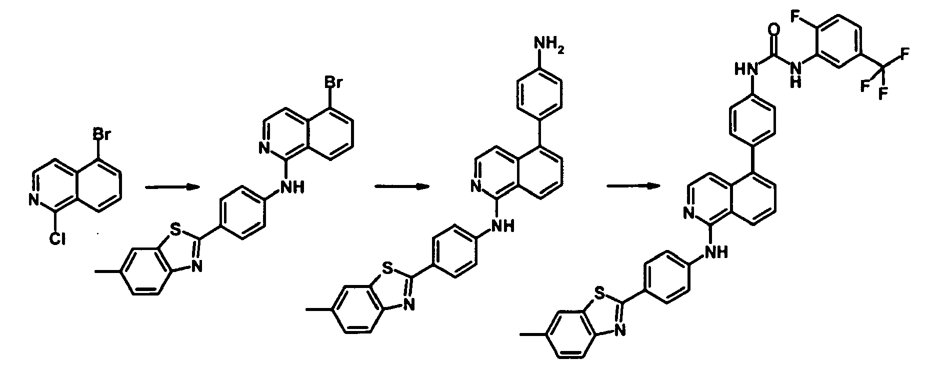

### a. 1-[4-(6-Methyl-benzothiazol-2-yl)-phenyl]amino-5-bromoisoquinoline

The title compound was prepared from 5-bromo-l-chloroisoquinoline and 4-(6-methyl-benzothiazol-2-yl)-phenylamine as described in example 4a.

### b. [5-(4-Amino-phenyl)-isoquinolin-1-yl]-[4-(6-methyl-benzothiazol-2-yl)-phenyl]-amine

The title compound was prepared from 1-[4-(6-methyl-benzothiazol-2-yl)-phenyl]amino-5-bromoisoquinoline and 4-(4,4,5,5-tetramethyl-[1,3,2]dioxaborolan-2-yl)aniline as described in example 1b.

### c. 1-(2-Fluoro-5-trifluoromethyl-phenyl)-3-(4-{1-[4-(6-methyl-benzothiazol-2-yl)-phenylamino]-isoquinolin-5-yl}-phenyl)-urea

The title compound was prepared from [5-(4-amino-phenyl)-isoquinolin-1-yl]-[4-(6-methyl-benzothiazol-2-yl)-phenyl]-amine as described in example 1d.

### Example 7.

### 1-(2-Fluoro-5-trifluoromethyl-phenyl)-3-(3-{1-[4-(6-methyl-benzothiazol-2-yl)-phenylamino]-isoquinolin-5-yl}-phenyl)-urea

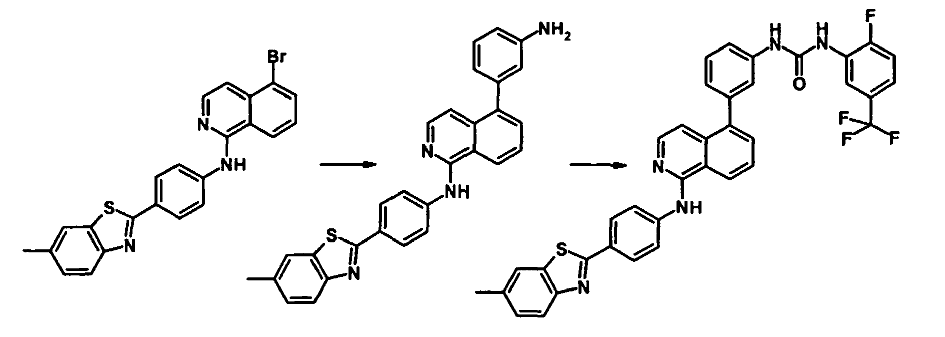

### a. [5-(3-Amino-phenyl)-isoguinolin-1-yl]-[4-(6-methyl-benzothiazol-2-yl)-phenyl]-amine

The title compound was prepared from 1-[4-(6-methyl-benzothiazol-2-yl)-phenyl]amino-5-bromoisoquinoline and 3-aminophenylboronic acid hydrochloride as described in example 1b.

### b. 1-(2-Fluoro-5-trifluoromethyl-phenyl)-3-(3-{1-[4-(6-methyl-benzothiazol-2-yl)-phenylamino]-isoquinolin-5-yl}-phenyl)-urea

The title compound was prepared from [5-(3-amino-phenyl)-isoquinolin-1-yl]-[4-(6-methyl-benzothiazol-2-yl)-phenyl]-amine as described in example 1d.

### Example 8.

### 1-(2-Fluoro-5-trifluoromethyl)-phenyl)-3-(4-isoquinolin-5-ylphenyl)urea

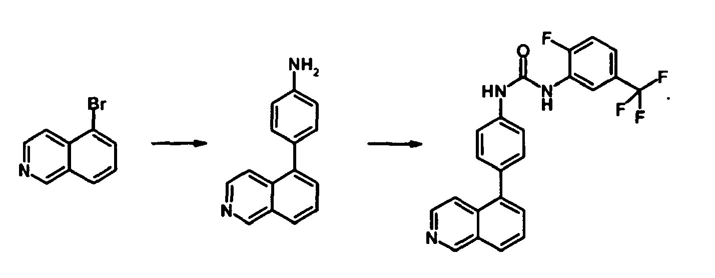

### a. 5-(4-Aminophenyl)isoquinoline

The title compound was prepared from 5-bromoisoquinoline and 4-(4,4,5,5-tetramethyl-[1,3,2]dioxaborolan-2-yl)aniline as described in example 1b.

### b. 1-(2-Fluoro-5-trifluoromethyl-phenyl)-3-(4-isoquinolin-5-ylphenyl)urea

The title compound was prepared from 5-(4-aminophenyl)isoquinoline as described in example 1d.

### Example 9.

### 1-Indan-5-yl-3-(3-isoguinolin-5-yl-phenyl)-urea

The title compound was prepared from 5-(4-aminophenyl)isoquinoline and 5-indanyl isocyanate as described in example 1d.

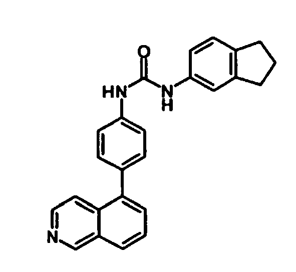

### Example 10.

### 1-(2-Fluoro-5-trifluoromethyl-phenyl)-3-{3-[1-(4-morpholin-4-yl-phenylamino)-isoquinolin-5-yl]-phenyl}-urea

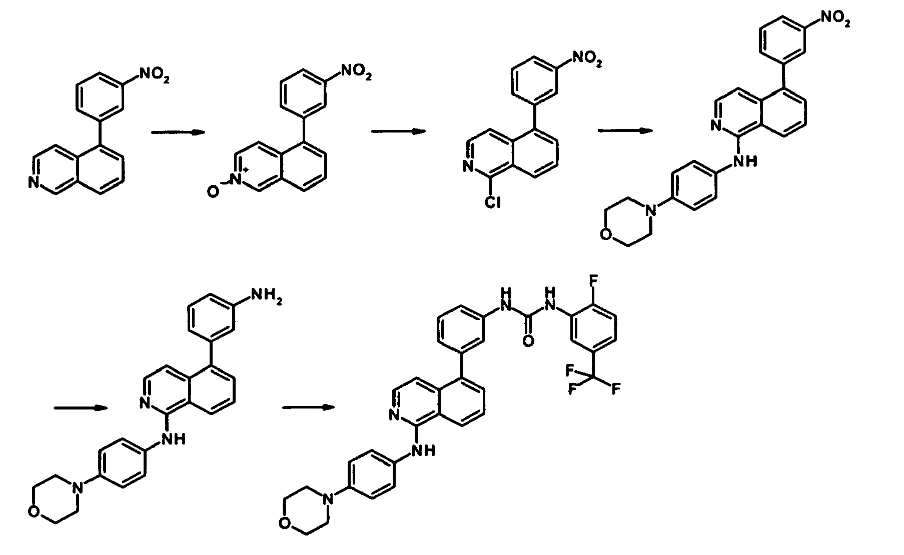

### a. 5-(3-Nitrophenyl)isoguinoline N-oxide

To a solution of peracetic acid (prepared from 64 mL of AcOH and 32 mL of 30% H₂O₂ 5-(3-nitrophenyl)isoquinoline (3.75 g, 15 mmol) was added then stirred at 80°C overnight. After cooling, the mixture was extracted with AcOEt. The organic layer was washed with water, NaHCO₃aq. NaHSO₃aq. And brine then dried over Na₂SO₄. After evaporation, the residue was purified on SiO₂ column chromatography and sequence recrystalisation from MeOH to give the title compound (yield not determined).

### b. 1-Chloro-5-(3-nitrophenyl)isoquinoline

The title compound was prepared from 5-(3-nitrophenyl)isoquinoline N-oxide as described in example 3b.

### c. 1-[4-(Morpholin-4-ylphenyl)amino]-5-(3-nitrophenyl)isoquinoline

The title compound was prepared from 1-Chloro-5-(3-nitrophenyl)isoquinoline and 4-morpholin-4-ylaniline as described in example 4a.

### d. 5-(3-Aminophenyl)-1-[4-(morpholin-4-ylphenyl)amino]isoquinoline

The title compound was prepared from 1-[4-(Morpholin-4-ylphenyl)amino]-5-(3-nitrophenyl)isoquinoline as described in example 1c.

### e. 1-(2-Fluoro-5-trifluoromethyl-phenyl)-3-{3-[1-(4-morpholin-4-yl-phenylamino)-isoquinolin-5-yl]-phenyl}-urea

The title compound was prepared from 5-(3-Aminophenyl)-1-[4-(morpholin-4-ylphenyl)amino]isoquinoline as described in example 1d.

### Example 11.

### 3-{5-[3-(3-Cyclohexyl-ureido)-phenyl]-isoquinolin-1-ylamino}-benzenesulfonamide

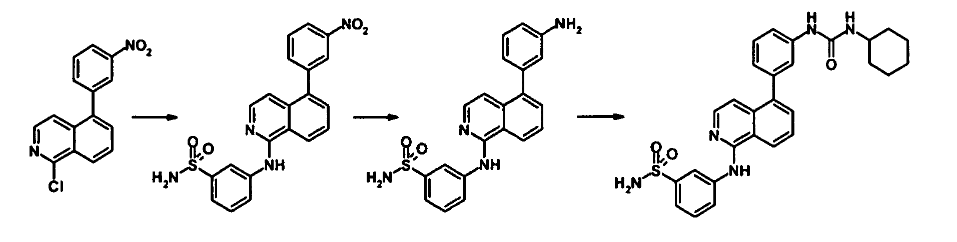

### a. 3-[5-(3-Nitrophenyl)isoguinolin-1-ylamino]benzenesulfonamide

The title compound was prepared from 1-Chloro-5-(3-nitrophenyl)isoquinoline and 3-aminobenzenesulfonamide as described in example 4a.

### b. 3-[5-(3-Aminophenyl)isoquinolin-1-ylamino]benzenesulfonamide

The title compound was prepared from 3-[5-(3-Nitrophenyl)isoquinolin-1-ylamino]benzenesulfonamide and 3-aminobenzenesulfonamide as described in example 1c.

### c. 3-{5-[3-(3-Cyclohexyl-ureido)-phenyl]-isoquinolin-1-ylamino]-benzenesulfonamide

The title compound was prepared from 3-[5-(3-aminophenyl)isoquinolin-1-ylamino]benzenesulfonamide and cyclohexyl isocyanate as described in example 1d.

### BIOLOGICAL DATA

### TIE-2 Enzyme assay (TIE2-E)

The TIE-2 enzyme assay used the LANCE method (Wallac) and GST-TIE2, baculovirus expressed recombinant constructs of the intracellular domains of human TIE2 (amino acids 762-1104, GenBank Accession # L06139) tagged by GST). The method measured the ability of the purified enzymes to catalyse the transfer of the γ-phosphate from ATP onto tyrosine residues in a biotinylated synthetic peptide, D1-15 (biotin-C6-LEARLVAYEGWVAGKKKamide). This peptide phosphorylation was detected using the following procedure: for enzyme preactivation, GST-TIE2 was incubated for 30mins at room temperature with 2 mM ATP, 5 mM MgCl2 and 12.5 mM DTT in 22.5 mM HEPES buffer (pH7.4). Preactivated GST-TIE2 was incubated for 30mins at room temperature in 96 well plates with 1 µM D1-15 peptide, 80 uM ATP, 10 mM MgCl2, 0.1 mg/ml BSA and the test compound (diluted from a 10 mM stock in DMSO, final DMSO concentration was 2.4%) in 1 mM HEPES (pH7.4). The reaction was stopped by the addition of EDTA (final concentration 45 mM). Streptavidin linked-APC (allophycocyanin, Molecular Probe) and Europium-labeled anti-phosphorylated tyrosine antibody (Wallac) were then added at the final concentration of 17 µg/well and 2.1 ug/well, respectively. The APC signal was measured using an ARVO multilabel counter. (Wallac Berthold Japan). The percent inhibition of activity was calculated relative to blank control wells. The concentration of test compound that inhibits 50% of activity (IC₅₀) was interpolated using nonlinear regression (Levernberg-Marquardt) and the equation, y = Vmax (1-x/(K+x)) + Y2, where "K" was equal to the IC₅₀. The IC₅₀ values were converted to plC₅₀ values, i.e., -log IC₅₀ in Molar concentration.

### VEGF-R2 enzyme assay (VEGF-E)

### Enzyme Assay:

Compounds of the present invention were tested for Vascular Endothelial Growth Factor 2 (VEGFR2) tyrosine kinase inhibitory activity in substrate phosphorylation assays. This assay examines the ability of small molecule organic compounds to inhibit the tyrosine phosphorylation of a peptide substrate.

### Homogenous Time-Resolved Fluorescence (HTRF) Assay

The substrate phosphorylation assays used the VEGFR2 catalytic domain, which was expressed in Sf9 insect cells as an amino-terminal GST-tagged fusion protein.
Autophosphorylation allows enzymes to be fully activated prior to addition to peptide substrates. The assays were performed using enzyme that had been activated by autophosphorylation via preincubation in buffer with ATP and magnesium. Activated enzyme is then diluted and added to titrated compound and the substrate mix.
200nM VEGFR2 enzyme was activated for 20 minutes at room temperature by incubating the enzyme in buffer containing 100mM HEPES (pH7.2), 75µM ATP, 0.3mM DTT, 0.1mg/mL BSA, and 10mM MgCl₂. After activation, VEGFR2. was diluted 100-fold into 2x dilution buffer: 200mM HEPES (pH 7.5), 0.2mg/mL BSA, 0.6mM DTT. 20µL of the diluted enzyme mix was added to 20µL of 2x substrate mix (150µM ATP, 20mM MgCl₂, 0.72µM biotinylated peptide) in the assay plates. Final assay conditions were: 100mM HEPES (pH 7.2), 75µM ATP, 10mM MgCl₂, 0.1mg/mL BSA, 0.3mM DTT, 0.36µM biotinylated peptide, and 1nM VEGFR2 enzyme. Assay plates were incubated for 1.5 hours at room temperature before the addition of 30µL 100mM EDTA to the wells to stop the enzymatic reaction. 40µL/well of HTRF mix were then added to the assay plates for the detection of phosphorylated substrate. Final assay concentrations were: 100mM HEPES (pH7.2), 0.1 mg/mL BSA, 15nM streptavidin-labeled allophycocyanin (PerkinElmer), and 1nM europium-labeled anti-phosphotyrosine antibody (PerkinElmer). Assay plates were left unsealed and were counted in a Wallac Multilabel Counter 1420 (PerkinElmer).
The data for dose responses were plotted as % Control calculated with the data reduction formula 100*(U1-C2)/(C1-C2) versus concentration of compound where U is the unknown value, C1 is the average control value obtained for DMSO, and C2 is the average control value obtained for 0.05M EDTA. Data were fitted to the curve described by: y = ((Vmax * x) / (K + x)) where Vmax is the upper asymptote and K is the IC₅₀. The results for each compound were recorded as plC₅₀ calculated as follows: plC50 = -Log10(K).

### EphB4 Enzyme Assay

The EphB4 enzyme assay used Scintillation Proximity Assay technology to measure enzyme activity. This method measured the ability of the purified enzymes to catalyse the transfer of the γ-phosphate from ATP onto tyrosine residues in a biotinylated synthetic peptide. The peptide used for the EphB4 enzyme activity assay was biotin-Ahx-MAHFENYEFFHAKKK-CONH2. (SEQ ID NO:1) The enzyme used was GST-EphB4, baculovirus expressed recombinant constructs of the intracellular domains of human EphB4 (amino acids 600-914, BR # 21454) tagged by GST. Peptide phosphorylation was detected using the following procedure: for enzyme preactivation, 7.4uM GST-EphB4 was incubated for 30mins at room temperature with 50 uM ATP and 10 mM MgCl₂ in 30 mM HEPES buffer (pH7.4). Preactivated GST-EphB4 was incubated then for 3 hours at room temperature in 96 well plates with 6 uM peptide, 1 uM ATP, 10 mM MgCl₂, 0.1 mg/ml BSA, 5 uCi/ml P33, 1 mM DTT, 1 mM CHAPS, 5mM KCI and 23-25 uM test compound in 100 mM HEPES (pH7.4). Each reaction was stopped by the addition of 0.1 mg Streptavidin SPA beads in 100mM EDTA/1x PBS, pH 7.2. The signal was measured using a Wallac Trilux scintillation counter (Wallac). The percent inhibition of activity was calculated relative to positive (C1) and negative (C2) control wells using, 100 * ( 1- ( U1 - C2 ) / ( C1 -C2 ) ). The concentration of test compound was determined using the equation, y = ((Vmax * x) / (K + x)) + Y2, where "K" was equal to the lC50. The lC50 values were converted to plC50 values, i.e., -log lC50 in Molar concentration.

The compounds of the Examples all demostrated inhibitory activity against at least one of the 3 kinases tested at a plC of > 5.0.

## Claims

1. A compound of Formula (I): 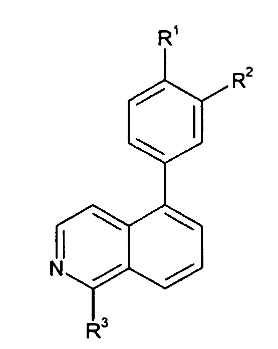 wherein:
One of R¹ and R² is H and the other represents - NHCONHR⁴
wherein R⁴ represents a phenyl or naphthyl group (which may be optionally substituted by one or more substituents independently selected from -C₁₋₆ alkyl, -C₁₋6 haloalkyl, - CH₂CH₂CH₂-, halogen, C₁-₆ alkoxy, C₁₋₆ haloalkoxy, OH, NO₂). C₃₋₇ cycloalkyl or R⁴ together with the NH to which it is bonded forms a morpholino group and
R³ is H or NHR⁵ wherein R⁵ is H, -quinolinyl or -isoquinolinyl, -(CONH)p phenyl (wherein p is 0 or 1 and the phenyl is optionally substituted by one or more substituents independently selected from halogen, -C₁₋₆ alkyl, -C₁₋₆ haloalkyl, - morpholino, -SO₂NH₂, benzothiazole (substituted by methyl))
or a salt or solvate thereof.

2. A compound according to claim 1 wherein R⁴ represents a phenyl group (which may be optionally substituted by one or more substituents selected from -C₁₋₆ haloalkyl, -CH₂CH₂CH₂-, halogen) or C₃₋₇ cycloalkyl.

3. A compound according to claims 1 - 2 wherein R³ is H or -NH R⁵ where in R⁵ is H, quinolinyl, -(CONH)p phenyl (wherein p is 0 or 1 and the phenyl is optionally substituted by one or more substituents independently selected from halogen, -C₁₋₆ haloalkyl -morpholino, -SO₂NH₂, benzothiazole, (substituted by methyl)).

4. A compound according to claims 1 - 3 of formula (1 a) 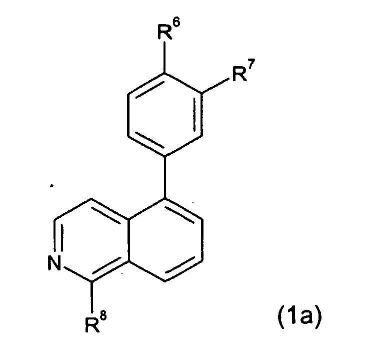
wherein one of R⁶ and R⁷ is H and the other represents -NHCONHR⁹;
R⁹ represents a phenyl group (which may be optionally substituted by one or more substituents independently selected from -C₁₋₆ haloalkyl, -CH₂CH₂CH₂-, halogen) or C₃₋₇ cycloalkyl;
R⁸ is H or NHR¹⁰;
R¹⁰ is H quinolinyl, -(CONH)p phenyl (where p is 0 or 1 and the phenyl is optionally substituted by one or more substituents independently selected from halogen, -C₁₋₆ haloalkyl, -morpholino, -SO₂NH₂, benzothiazole (substituted by methyl)).

5. A compound according to claim 4 wherein NHCONHR⁹ represents

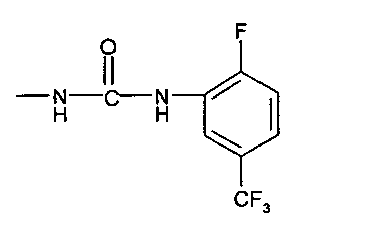

6. A compound according to claim 4 and 5 where in R¹⁰ is H,

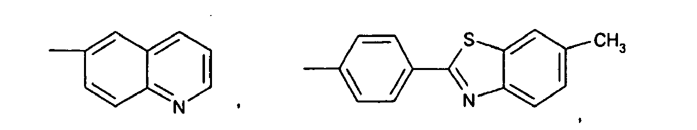

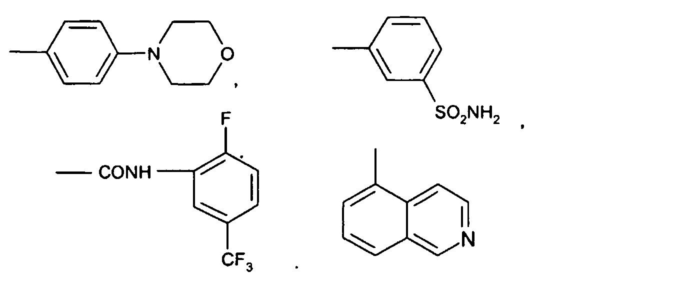

7. A compound as claimed in claim 1 - 6, selected from the group consisting of:
1-(2-Fluoro-5-trifluoromethyl-phenyl)-3-(3-isoquinolin-5-ylphenyl)urea;
1-Cyclohexyl-3-(3-isoquinolin-5-ylphenyl)urea;
1-[3-(1-Amino-isoquinolin-5-yl)-phenyl]-3-(2-fluoro-5-trifluoromethyl-phenyl)-urea ;
1-(2-fluoro-5-trifluoromethyl-phenyl)-3-(5-{3-[3-(2-fluoro-5-trifluoromethyl-phenyl)-ureido]-phenyl}-isoquinolin-1-yl)-urea;
1-(2-Fluoro-5-trifluoromethyl-phenyl)-3-{3-[1-(quinolin-6-ylamino)-isoquinolin-5-yl]-phenyl}-urea;
1-(2-Fluoro-5-trifluoromethyl-phenyl)-3-(4-{1-[4-(6-methyl-benzothiazol-2-yl)-phenylamino]-isoquinolin-5-yl}-phenyl)-urea;
1-(2-Fluoro-5-trifluoromethyl-phenyl)-3-(3-{1-[4-(6-methyl-benzothiazol-2-yl)-phenylamino]-isoquinolin-5-yl}-phenyl)-urea;
1-(2-Fluoro-5-trifluoromethyl-phenyl)-3-(4-isoquinolin-5-ylphenyl)urea;
1 -indan-5-yl-3-(3-isoquinolin-5-yi-phenyl)-urea;
1-(2-Fluoro-5-trifluoromethyl-phenyl)-3-{3-[1-(4-morpholin-4-yl-phenylamino)-isoquinolin-5-yl]-phenyl}-urea;
3-{5-[3-(3-Cyclohexyl-ureido)-phenyl]-isoquinolin-1-ylamino}-benzenesulfonamide;
or a salt or solvate, thereof.

8. A pharmaceutical composition, comprising: a therapeutically effective amount of a compound as claimed in any one of claims 1 - 7, or a salt or a solvate thereof and one or more of pharmaceutically acceptable carriers, diluents and excipients.

9. A pharmaceutical composition according to claim 8 further comprising an agent to inhibit growth factor receptor function

10. A compound as claimed in any of claims 1 - 7, or a salt or a solvate thereof for use in therapy.

11. The use of a compound according to claims 1 - 7 or a salt or a solvate thereof in the manufacture of a medicament for the treatment of inappropriate angiogenesis.

## Patentansprüche

1. Verbindung der Formel (I): 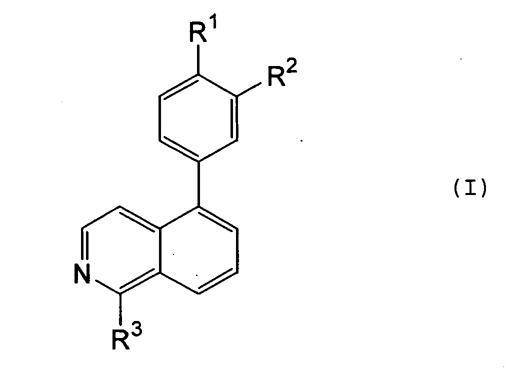 worin:
eines von R¹ und R² H ist und das andere -NHCONHR⁴ darstellt,
worin R⁴ eine Phenyl- oder Naphthylgruppe (die gegebenenfalls mit einem oder mehreren Substituenten substituiert sein kann, die unabhängig ausgewählt sind aus -C₁₋₆-Alkyl, -C₁₋₆-Halogenalkyl, -CH₂CH₂CH₂-, Halogen, C₁₋₆-Alkoxy, C₁₋₆-Halogenalkoxy, OH, NO₂) oder C₃₋₇-Cycloalkyl darstellt, oder R⁴ zusammen mit dem NH, an das es gebunden ist, eine Morpholinogruppe bildet, und
R³ H oder NHR⁵ ist, worin R⁵ H, -Chinolinyl oder -Isochinolinyl, -(CONH)ₚ-phenyl (worin p 0 oder 1 ist und das Phenyl gegebenenfalls mit einem oder mehreren Substituenten substituiert ist, die unabhängig ausgewählt sind aus Halogen, -C₁₋₆-Alkyl, -C₁₋₆-Halogenalkyl, -Morpholino, -SO₂NH₂, Benzothiazol (mit Methyl substituiert)) ist,
oder ein Salz oder Solvat davon.

2. Verbindung gemäß Anspruch 1, worin R⁴ eine Phenylgruppe (die gegebenenfalls mit einem oder mehreren Substituenten substituiert sein kann, die ausgewählt sind aus -C₁₋₆-Halogenalkyl, -CH₂CH₂CH₂-, Halogen) oder C₃₋₇-Cycloalkyl darstellt.

3. Verbindung gemäß den Ansprüchen 1 bis 2, worin R³ H oder -NHR⁵ ist, worin R⁵ H, Chinolinyl, -(CONH)ₚ-phenyl (worin p 0 oder 1 ist und das Phenyl gegebenenfalls mit einem oder mehreren Substituenten substituiert ist, die unabhängig ausgewählt sind aus Halogen, -C₁-₆-Halogenalkyl, -Morpholino, -SO₂NH₂, Benzothiazol (mit Methyl substituiert)) ist.

4. Verbindung gemäß den Ansprüchen 1 bis 3 der Formel (1a): 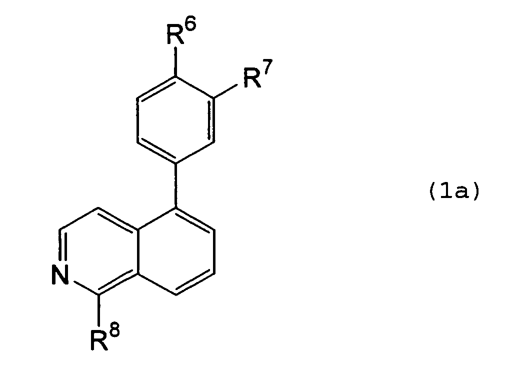
worin eines von R⁶ und R⁷ H ist und das andere -NHCONHR⁹ darstellt;
R⁹ eine Phenylgruppe (die gegebenenfalls mit einem oder mehreren Substituenten substituiert sein kann, die unabhängig ausgewählt sind aus -C₁₋₆-Halogenalkyl, -CH₂CH₂CH₂-, Halogen) oder C₃₋₇-Cycloalkyl darstellt;
R⁸ H oder NHR¹⁰ ist;
R¹⁰ H, Chinolinyl, -(CONH)ₚ-phenyl (worin p 0 oder 1 ist und das Phenyl gegebenenfalls mit einem oder mehreren Substituenten substituiert ist, die unabhängig ausgewählt sind aus Halogen, -C₁₋₆-Halogenalkyl, -Morpholino, -SO₂NH₂, Benzothiazol (mit Methyl substituiert)) ist.

5. Verbindung gemäß Anspruch 4, worin NHCONHR⁹ 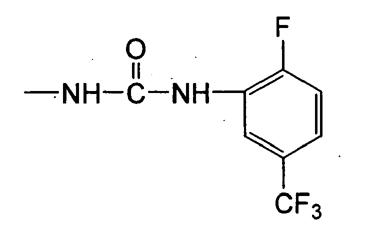 darstellt.

6. Verbindung gemäß den Ansprüchen 4 und 5, worin R¹⁰ H, 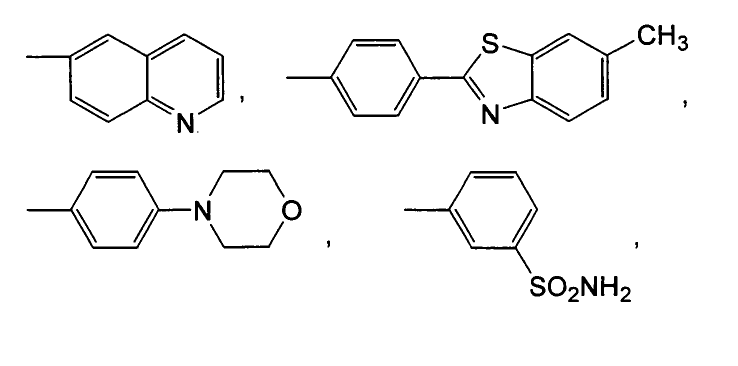 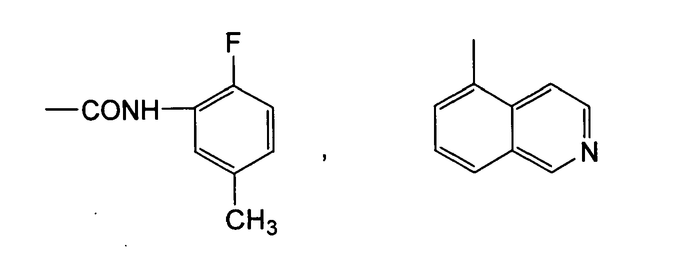 ist.

7. Verbindung gemäß den Ansprüchen 1 bis 6, die aus der Gruppe ausgewählt ist, die aus folgendem besteht:
1-(2-Fluor-5-trifluormethyl-phenyl)-3-(3-isochinolin-5-ylphenyl)harnstoff,
1-Cyclohexyl-3-(3-isochinolin-5-ylphenyl)harnstoff,
1-[3-(1-Amino-isochinolin-5-yl)phenyl]-3-(2-fluor-5-trifluormethyl-phenyl)harnstoff,
1-(2-Fluor-5-trifluormethyl-phenyl)-3-(5-{3-[3-(2-fluor-5-trifluormethyl-phenyl)-ureido]-phenyl}-isochinolin-1-yl)harnstoff,
1-(2-Fluor-5-trifluormethyl-phenyl)-3-(3-[1-(chinolin-6-ylamino)-isochinolin-5-yl]-phenyl}harnstoff,
1-(2-Fluor-5-trifluormethyl-phenyl)-3-(4-{1-[4-(6-methyl-benzothiazol-2-yl)-phenylamino]-isochinolin-5-yl}-phenyl)harnstoff,
1-(2-Fluor-5-trifluormethyl-phenyl)-3-(3-{1-[4-(6-methyl-benzothiazol-2-yl)-phenylamino]-isochinolin-5-yl}-phenyl)harnstoff,
1-(2-Fluor-5-trifluormethyl-phenyl)-3-(4-isochinolin-5-ylphenyl)harnstoff,
1-Indan-5-yl-3-(3-isochinolin-5-ylphenyl)harnstoff,
1-(2-Fluor-5-trifluormethyl-phenyl)-3-{3-[1-(4-morpholin-4-yl-phenylamino)-isochinolin-5-yl]-phenyl}harnstoff und
3-{5-[3-(3-Cyclohexyl-ureido)phenyl]-isochinolin-1-ylamino}benzolsulfonamid,
oder ein Salz oder Solvat davon.

8. Pharmazeutische Zusammensetzung, die folgendes umfaßt: eine therapeutisch effektive Menge einer Verbindung gemäß einem der Ansprüche 1 bis 7 oder eines Salzes oder Solvats davon und eines oder mehrere aus pharmazeutisch annehmbaren Trägern, Verdünnungsstoffen und Exzipienten.

9. Pharmazeutische Zusammensetzung gemäß Anspruch 8, die ferner ein Mittel zur Inhibierung einer Wachstumsfaktor-Rezeptorfunktion umfaßt.

10. Verbindung gemäß einem der Ansprüche 1 bis 7 oder ein Salz oder Solvat davon zur Verwendung in der Therapie.

11. Verwendung einer Verbindung gemäß den Ansprüchen 1 bis 7 oder eines Salzes oder Solvats davon bei der Herstellung eines Medikaments zur Behandlung einer unangemessenen Angiogenese.

## Revendications

1. Composé de Formule (I) : 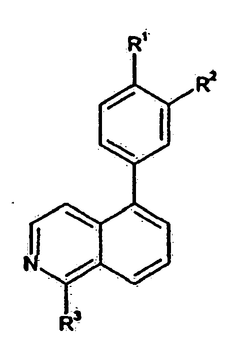 dans laquelle :
l'un des R¹ et R² est un atome d'hydrogène et l'autre représente -NHCONHR⁴ où R⁴ représente un groupe phényle ou naphtyle (qui peut être éventuellement substitué par un ou plusieurs substituants choisis indépendamment parmi un groupe -alkyle en C₁₋₆, -haloalkyle en C₁₋₆, -CH₂CH₂CH₂-, un atome d'halogène, un groupe alcoxy en C₁₋₆, haloalcoxy en C₁₋₆, OH, NO₂), cycloalkyle en C₃₋₇ ou bien R⁴ forme un groupe morpholino avec le groupe NH auquel il est lié et
R³ est un atome d'hydrogène ou NHR⁵ où R⁵ est un atome d'hydrogène, un groupe -quinoléinyle ou -isoquinoléinyle, -(CONH)ₚphényle (où p est 0 ou 1 et le groupe phényle est éventuellement substitué par un ou plusieurs substituants choisis indépendamment parmi un atome d'halogène, un groupe -alkyle en C₁₋₆, -haloalkyle en C₁₋₆, -morpholino, -SO₂NH₂, benzothiazole (substitué par un groupe méthyle))
ou un sel ou un solvate de celui-ci.

2. Composé selon la revendication 1, dans lequel R⁴ représente un groupe phényle (qui peut être éventuellement substitué par un ou plusieurs substituants choisis parmi un groupe -haloalkyle en C₁₋₆, -CH₂CH₂CH₂-, un atome d'halogène) ou un groupe cycloalkyle en C₃₋₇.

3. Composé selon les revendications 1 à 2, dans lequel R³ est un atome d'hydrogène ou -NHR⁵, où R⁵ est un atome d'hydrogène, un groupe -quinoléinyle, -(CONH)ₚphényle (où p est 0 ou 1 et le groupe phényle est éventuellement substitué par un ou plusieurs substituants choisis indépendamment parmi un atome d'halogène, un groupe -haloalkyle en C₁₋₆, -morpholino, -SO₂NH₂, benzothiazole (substitué par un groupe méthyle)).

4. Composé selon les revendications 1 à 3 de formule (1a) 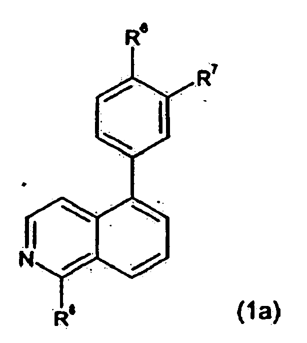 dans laquelle
l'un des R⁶ et R⁷ est un atome d'hydrogène et l'autre représente -NHCONHR⁹ ; dans lequel R⁹ représente un groupe phényle (qui peut être éventuellement substitué par un ou plusieurs substituants choisis indépendamment parmi un groupe -haloalkyle en C₁₋₆, -CH₂CH₂CH₂-, un atome d'halogène), ou un groupe cycloalkyle en C₃₋₇ ;
R⁸ est un atome d'hydrogène ou NHR¹⁰ ;
R¹⁰ est un atome d'hydrogène, un groupe -quinoléinyle, -(CONH)ₚphényle (où p est 0 ou 1 et le groupe phényle est éventuellement substitué par un ou plusieurs substituants choisis indépendamment parmi un atome d'halogène, un groupe -haloalkyle en C₁₋₆, -morpholino, -SO₂NH₂, benzothiazole (substitué par un groupe méthyle)).

5. Composé selon la revendication 4, dans lequel NHCONHR⁹ représente

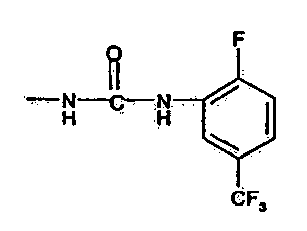

6. Composé selon les revendications 4 et 5, dans lequel R¹⁰ est un atome d'hydrogène.

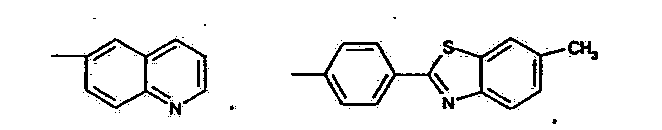

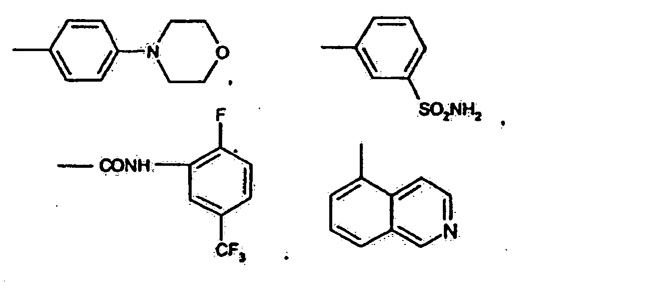

7. Composé selon les revendications 1 à 6, choisi dans le groupe consistant en :
1-(2-fluoro-5-trifluorométhyl-phényl)-3-(3-isoquinoléin-5-yl-phényl)urée ;
1-cyclohexyl-3-(3-isoquinoléin-5-ylphényl)urée ;
1-[3-(1-amino-isoquinoléin-5-yl)phényl]-3-(2-fluoro-5-trifluorométhyl-phényl)urée ;
1-(2-fluoro-5-trifluorométhyl-phényl)-3-(5-{3-[3-(2-fluoro-5-trifluorométhyl-phényl)-uréido]-phényl}-isoquinoléin-1-yl)urée ;
1-(2-fluoro-5-trifluorométhyl-phényl)-3-{3-[1-(quinoléin-6-ylamino)-isoquinoléin-5-yl]-phényl}urée ;
1-(2-fluoro-5-trifluorométhyl-phényl)-3-(4-{1-[4-(6-méthyl-benzothiazol-2-yl)-phénylamino]-isoquinoléin-5-yl}-phényl)urée ;
1-(2-fluoro-5-trifluorométhyl-phényl)-3-(3-{1-[4-(6-méthyl-benzothiazol-2-yl)-phénylamino]-isoquinoléin-5-yl}-phényl)urée ;
1-(2-fluoro-5-trifluorométhyl-phényl)-3-(4-isoquinoléin-5-yl-phényl)urée ;
1-indan-5-yl-3-(3-isoquinoléin-5-yl-phényl)urée ;
1-(2-fluoro-5-trifluorométhyl-phényl)-3-{3-[1-(4-morpholin-4-yl-phénylamino)-isoquinoléin-5-yl]phényl)urée ;
3-{5-[3-(3-cyclohexyl-uréido)-phényl]-isoquinoléin-1-ylamino}-benzènesulfonamide ;
ou un sel ou un solvate de ceux-ci.

8. Composition pharmaceutique comprenant une quantité efficace du point de vue thérapeutique d'un composé selon l'une quelconque des revendications 1 à 7, ou d'un sel ou d'un solvate de celui-ci et un ou plusieurs supports, diluants et excipients acceptables du point de vue pharmaceutique.

9. Composition pharmaceutique selon la revendication 8 comprenant de plus un agent destiné à inhiber la fonction de récepteurs de facteurs de croissance

10. Composé selon l'une quelconque des revendications 1 à 7, ou un sel ou un solvate de celui-ci utile en thérapie.

11. Utilisation d'un composé selon les revendications 1 à 7 ou d'un sel ou d'un solvate de celui-ci dans la fabrication d'un médicament destiné au traitement d'une angiogenèse inappropriée.
